(19) Europäisches Patentamt
European Patent Office
Office européen des brevets

(11) Veröffentlichungsnummer: **0 009 709**
**B1**

(12) # EUROPÄISCHE PATENTSCHRIFT

(45) Veröffentlichungstag der Patentschrift: **15.02.84**

(51) Int. Cl.³: **C 07 C 69/743,** A 01 N 53/00

(21) Anmeldenummer: **79103488.7**

(22) Anmeldetag: **17.09.79**

(54) **Fluoralkenylsubstituierte Cyclopropancarbonsäureester (I), Verfahren zu ihrer Herstellung, hierbei erhaltene Zwischenprodukte und deren Herstellung, die Ester (I) enthaltende Mittel und die Verwendung der Ester (I) zur Bekämpfung von Insekten und/oder Spinnentieren.**

(30) Priorität: **29.09.78 DE 2842541**

(43) Veröffentlichungstag der Anmeldung:
**16.04.80 Patentblatt 80/8**

(45) Bekanntmachung des Hinweises auf die Patenterteilung:
**15.02.84 Patentblatt 84/7**

(84) Benannte Vertragsstaaten:
**AT BE CH DE FR GB IT NL SE**

(56) Entgegenhaltungen:
**EP-A-0 003 336**
**DE-A-2 547 534**
**DE-A-2 802 962**
**GB-A-2 000 764**

(73) Patentinhaber: **BAYER AG, Zentralbereich Patente, Marken und Lizenzen, D-5090 Leverkusen 1, Bayerwerk (DE)**

(72) Erfinder: **Lantzsch, Reinhard, Dr., Heymannstrasse 32, D-5090 Leverkusen 1 (DE)**
Erfinder: **Hagemann, Hermann, Dr., Roggendorfstrasse 55, D-5000 Köln 80 (DE)**
Erfinder: **Arlt, Dieter, Dr., Rybniker Strasse 2, D-5000 Köln 80 (DE)**
Erfinder: **Jautelat, Manfred, Dr., Müllersbaum 28, D-5093 Burscheid 1 (DE)**
Erfinder: **Hammann, Ingeborg, Dr., Belfortstrasse 9, D-5000 Köln 1 (DE)**
Erfinder: **Behrenz, Wolfgang, Dr., Untergründemich 14, D-5063 Overath (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Fluoralkenylsubstituente Cyclopropancarbonsäureester (I), Verfahren zu ihrer Herstellung, hierbei erhaltene Zwischenprodukte und deren Herstellung, die Ester (I) enthaltende Mittel und die Verwendung der Ester (I) zur Bekämpfung von Insekten und/oder Spinnentieren

Die vorliegende Erfindung betrifft neue fluoralkenylsubstituierte Cyclopropancarbonsäureester, Verfahren zu ihrer Herstellung, ihre Verwendung als Insektizide sowie neue Zwischenprodukte zur Herstellung dieser Wirkstoffe.

Fluoralkenylsubstituierte Cyclopropancarbonsäureester sind bereits bekannt aus der deutschen Offenlegungsschrift 2802962.

Diese daraus bekannten Verbindungen besitzen jedoch den Nachteil zu geringer Wirksamkeit. Vor allem bei niedrigen Aufwandkonzentrationen ist Wirkung, Wirkungsdauer und Wirkungsspektrum nicht befriedigend.

Die neuen fluoralkenylsubstituierten Cyclopropancarbonsäureester lassen sich zudem preisgünstiger herstellen, da die fluorhaltigen Ausgangsstoffe leichter zugänglich sind.

Weitere fluoralkenylsubstituierte Cyclopropancarbonsäureester sind in EP-A3336 beschrieben.

1. Es wurden die neuen fluoralkenylsubstituierten Cyclopropancarbonsäureester der Formel I gefunden

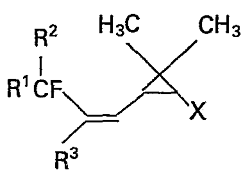

(I)

in welcher

$R^1$ für Chlor,

$R^2$ für Chlor,

$R^3$ für Cl oder Br,

O

X für -C-OR steht und

R für $C_{1-4}$-Alkyl sowie für Reste der Formeln

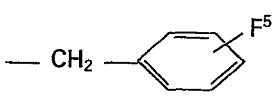

oder für den Fall, dass $R^3$ für Cl steht, für:

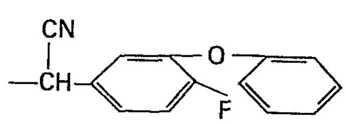

steht.

2. Es wurde ferner gefunden, dass man die neuen fluoralkenylsubstituierten Cyclopropancarbonsäureester der Formel I erhält, indem man eine Säure oder deren reaktionsfähiges Derivat der Formel II

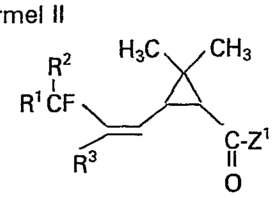

(II)

in welcher

$R^1$, $R^2$ und $R^3$ die unter 1. (oben) angegebene Bedeutung haben und

$Z^1$ Halogen, vorzugsweise Chlor, oder OH bedeutet,

mit einem Alkohol oder dessen reaktionsfähigem Derivat der Formel III

$$R - Z^2 \qquad (III)$$

in welcher

R die unter 1.(oben) angegebene Bedeutung hat und

$Z^2$ OH, Cl oder Br bedeutet,

gegebenenfalls in Gegenwart von Lösungsmitteln, Säureakzeptoren und/oder Phasentransferkatalysatoren umsetzt.

3. Es wurden ferner die neuen Säuren, bzw. die reaktionsfähigen Derivate derselben, der Formel II gefunden,

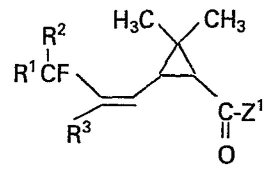

(II)

in welcher

$R^1$, $R^2$, $R^3$ und $Z^1$ die unter 2. (oben) angegebene Bedeutung besitzen.

4. Es wurde ferner gefunden, dass man die neuen Säuren, bzw. die reaktionsfähigen Derivate derselben der Formel II erhält, indem man die Verbindungen der Formel IV

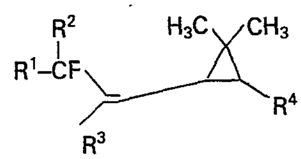

(IV)

in welcher

$R^4$ für -$COOC_{1-4}$-Alkyl, CN oder -$COCH_3$ steht und

$R^1$, $R^2$ und $R^3$ die oben angegebene Bedeutung besitzen

für den Fall, dass $R^4$ für -CO-$C_{1-4}$-Alkyl oder CN steht verseift oder für den Fall, dass $R^4$ für -C-$CH_3$ steht, oxidiert und gegebenenfalls die Säure, in welcher $R^4$ COOH bedeutet mit einem Halogenierungsmittel umsetzt.

5. Die Verbindungen der Formel IV sind neu:

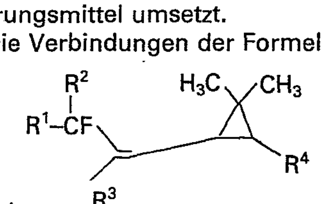

(IV)

in welcher

$R^1$, $R^2$ und $R^3$ die unter 1. (oben) angegebene Bedeutung besitzen und

$R^4$ für -CN, -C-$CH^3$ oder -CO-$C_{1-4}$-Alkyl steht.

6. Die neuen Verbindungen der Formel IV erhält man, indem man die Verbindungen der Formel V

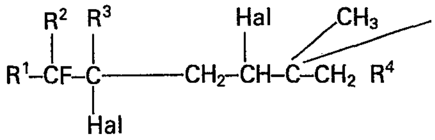

(V)

in welcher
$R^1$, $R^2$, $R^3$ und $R^4$ die unter 4. (oben) angegebene Bedeutung besitzen, und
Hal für Halogen, vorzugsweise für Chlor oder Brom steht und gleich oder verschieden sein kann,
dehydrohalogeniert.

7. Die Verbindungen der Formel V sind neu:

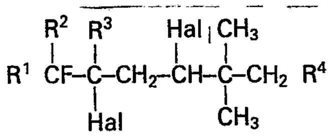

(V)

in welcher
$R^1$, $R^2$, $R^3$ und $R^4$ die unter 5. (oben) und
Hal die unter 6. (oben) angegebene Bedeutung besitzen.

8. Die neuen Verbindungen der Formel V erhält man, indem man die Halogenverbindungen der Formel VI

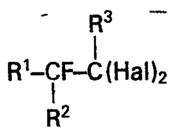

(VI)

in welcher
Hal gleich oder verschieden ist und Halogen, vorzugsweise Chlor oder Brom bedeutet und
$R^1$, $R^2$ und $R^3$ die unter 1. (oben) angegebene Bedeutung besitzt,
an Olefine der Formel VII

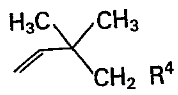

(VII)

in welcher
$R^4$ die unter 5. (oben) angegebene Bedeutung besitzt, gegebenenfalls in Gegenwart eines Katalysator, addiert.

Die Verbindungen der Formel I gemäss 1. (oben) zeigen gute insektizide Eigenschaften.

Überraschenderweise zeigen die neuen erfindungsgemässen Wirkstoffe gemäss 1. (oben) eine erheblich höhere Wirksamkeit als die aus dem Stand der Technik bekannten Verbindungen.

Von den erfindungsgemässen Verbindungen der Formel I gemäss 1. (oben) sind diejenigen bevorzugt, in denen
$R^3$ für Cl steht und
R für einen der folgenden Reste steht.

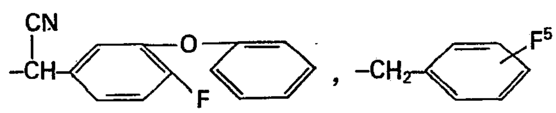

Die Herstellung der erfindungsgemässen fluoralkenylsubstituierten Cyclopropancarbonsäureester kann durch folgendes Reaktionsschema wiedergegeben werden:

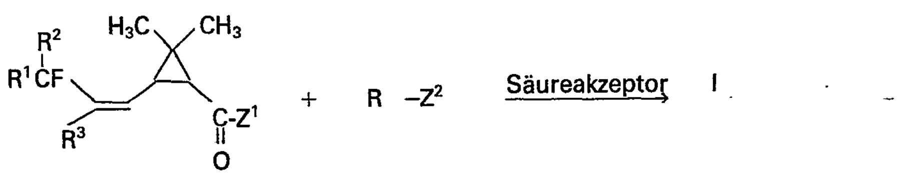


Verwendet man beispielsweise gemäss Verfahren 2. (oben) 2,2-Dimethyl-3-(2',3',3'-trichlor-3'-fluorpropenyl)-cycloropancarbonsäurechlorid und 3-Phenoxy-4-fluor-α-cyanobenzylalkohol als Ausgangsmaterialien, so kann der Reaktionsverlauf durch das folgende Formelschema wiedergegeben werden:

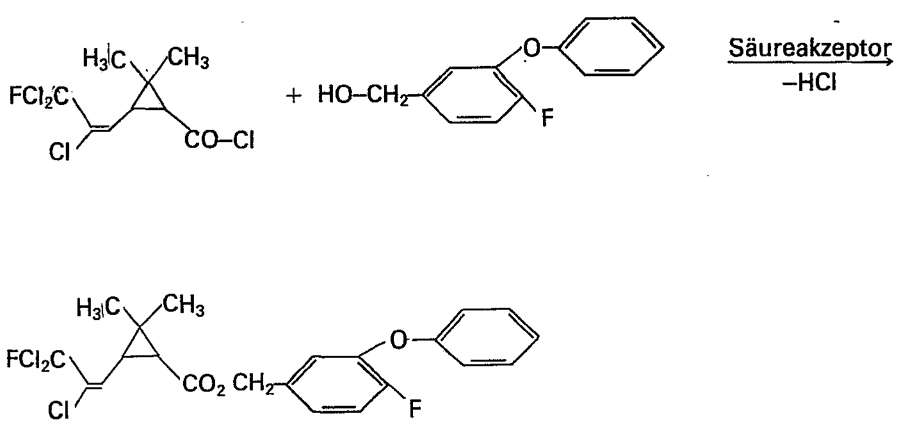


Die als Ausgangsprodukte zu verwendenden Cyclopropancarbonsäuren bzw. deren reaktionsfähige Derivate der Formel II sind neu. Als reaktionsfähige Derivate werden bevorzugt die Säurechloride eingesetzt.

Als Beispiele für die als Ausgangsprodukte zu verwendenden Verbindungen der Formel II seien im einzelnen genannt:

2,2-Dimethyl-3-(2',3',3'-trichlor-3'-fluorpenyl)-cyclopropancarbonsäure (und -chlorid).

Die neuen Verbindungen der Formel II können nach dem unter 4. (oben) angegebenen Verfahren hergestellt werden (Einzelheiten siehe weiter unten).

Die ebenfalls als Ausgangsprodukte zu verwendenden Alkohols bzw. reaktionsfähigen Derivate der Formel III sind bekannt und nach allgemein üblichen, in der Literatur beschriebenen Verfahren herstellbar (vgl. z.B. DT-AS oder DT-OS 25 54 883, 19 26 433, 26 12 115, 26 15 435, 24 36 178, 24 36 462 sowie Monatshefte 67, Seite 35 [1936]).

Bevorzugt sind Verbindungen der Formel III in welcher R die weiter oben angegebene bevorzugte oder besonders bevorzugte Bedeutung hat.

Als Beispiele für die als Ausgangsprodukte zu verwendenden Verbindungen der Formel III seien im einzelnen genannt:

Pentafluorbenzylalkohol

3- Phenoxy-4-fluor-α-cyanobenzylalkohol

Pentafluorbenzylchlorid.

Zur Herstellung der erfindungsgemässen Verbindungen der Formel I gemäss 1. (oben) aus Carbonsäuren bzw. Carbonsäurehalogeniden der Formel II und Alkoholen bzw. Chloriden oder Bromiden der Formel III können als Säureakzeptoren alle üblichen Säurebindemittel Verwendung finden.

Besonders bewährt haben sich Alkalihydroxide, -carbonate und -alkoholate, wie Kaliumhydroxid, Natriumhydroxid, Natriummethylat, Kaliumcarbonat, Natriumäthylat, ferner aliphatische, aromatische oder heterocyclische Amine, beispielsweise Triäthylamin, Trimethylamin, Dimethylanilin, Dimethylbenzylamin und Pyridin.

Die Reaktionstemperatur kann innerhalb eines grösseren Bereichs variiert werden. Im allgemeinen arbeitet man bei der Umsetzung der Säurehalogenide mit Alkoholen zwischen 0 und 100°C, vorzugsweise bei 15 bis 40°C und bei der Umsetzung der Carbonsäure mit den Halogeniden zwischen 50 und 150°C, vorzugsweise bei 80 bis 120°C. Im letzteren Falle wird bevorzugt in Gegenwart eines Katalysators gearbeitet.

Als Katalysatoren kommen alle sogenannten Phasentransferkatalysatoren in Betracht, wie beispielsweise Kronenäther oder quartäre Ammonium- oder Phosphoniumsalze. Bevorzugt sind quartäre Ammoniumsalze, wie beispielsweise Tetrabutylammoniumchlorid, Tetrabutylammoniumbromid, Benzyltriäthylammoniumchlorid oder Methyltrioctylammoniumchlorid.

Die Umsetzung lässt man im allgemeinen bei Normaldruck ablaufen. Das Verfahren zur Herstellung der erfindungsgemässen Verbindung wird bevorzugt unter Mitverwendung geeigneter Lösung- und Verdünnungsmittel durchgeführt. Als solche kommen praktisch alle inerten organischen Solventien in Frage. Hierzu gehören insbesondere aliphatische und aromatische, gegebenenfalls chlorierte Kohlenwasserstoffe wie Benzol, Toluol, Xylol, Benzin, Methylenchlorid, Chloroform, Dichloräthan, Chlorbenzol, o-Dichlorbenzol oder Äther, z.B. Diäthyl-, Diisopropyl oder Dibutyläther, ausserdem Nitrile, wie Aceto- und Propionitril.

Zur Durchführung des Verfahrens setzt man die Ausgangskomponenten vorzugsweise in äquimolaren Verhältnissen ein. Die Reaktionskomponenten werden im allgemeinen in einem der angegebenen Lösungsmittel zusammengegeben und meist bei erhöhter Temperatur nach Zugabe des Säureakzeptors und gegebenenfalls des Katalysators zur Vervollständigung der Reaktion eine oder mehrere Stunden gerührt. Anschliessend giesst man das Reaktionsgemisch in Wasser, trennt die organische Phase ab und wäscht diese mit Wasser neutral. Nach dem Trocknen wird das Lösungsmittel im Vakuum abdestilliert. Die neuen Verbindungen fallen in Form von Ölen an, die sich zum Teil nicht unzersetzt destillieren lassen, jedoch durch sogenanntes «Andestillieren», d.h. durch längeres Erhitzen unter vermindertem Druck auf mässig erhöhte Temperaturen von den letzten flüchtigen Anteilen befreit und auf diese Weise gereinigt werden. Zu ihrer Charakterisierung dient der Brechnungsindex.

Die neuen Säuren bzw. Säurehalogenide der Formel II lassen sich nach dem unter 4. (oben) angegebenen Verfahren aus den Verbindungen der Formel IV herstellen.

O

Bei Variante 4a wird für den Fall, dass R für -CO-$C_{1-4}$-Alkyl steht in an sich üblicher Weise sauer oder alkalisch verseift. Bevorzugt ist die alkalische Verseifung.

Für den Fall, dass R für CN steht, wird die Nitrilgruppe in eine Estergruppe umgewandelt, indem man das Nitril in dem entsprechenden Alkohol $C_{1-4}$-Alkyl-OH löst und die Lösung mit Chlorwasserstoff sättigt, üblicherweise bei etwa 0 bis 20°C. Nach einigem Stehen bildet sich dabei das Imidchlorid, aus dem mit Wasser der Ester entsteht.

Für die Verseifung kommen alle üblichen Verseifungsmittel in Frage, wie beispielsweise Schwefelsäure, Essigsäure oder Alkalilaugen. Besonders bevorzugt ist KOH in alkoholischer Lösung, z.B. in Methanol.

Die Verseifung kann bei erhöhter Temperatur durchgeführt werden, beispielsweise zwischen 20 und 100°C, bevorzugt wird jedoch bei 20 bis 40°C gearbeitet.

Die Säuren werden durch Ansäuern mit wässrigen Säuren in Freiheit gesetzt und durch Extraktion von den Salzen abgetrennt, sowie gegebenenfalls durch Destillation gereinigt.

Die Säurehalogenide können nach den üblichen Methoden mit den üblichen Halogenierungsmitteln, wie beispielsweise $SOCl_2$, $COCl_2$, $PCl_3$, $PCl_5$, Oxalylchlorid oder -bromid, $PBr_3$, hergestellt werden.

O

Bei Variante 4b steht R für -C-$CH_3$. Man erhält die Säure durch Oxidation, vorzugsweise mit Hypochlorit (Chlorlauge) oder Hypobromit. Beispiele

für solche Haloformreagentien sind Natriumhypochlorit, Kaliumhypochlorit, Calziumhypochlorit, Natriumhypobromit, Kaliumhypobromit und Calziumhypobromit. Ferner kann die Reaktion auch durchgeführt werden, indem man ein Halogen in eine die Ausgangsverbindung enthaltende wässrige Alkalihydroxidlösung einleitet. Die Reaktionstemperatur beträgt im allgemeinen –20 bis 100°C, vorzugsweise 0 bis 70°C. Im allgemeinen wird Wasser als Lösungsmittel verwendet, es können aber auch organische Lösungsmittel als Lösungsvermittler zugesetzt werden. Durch Zusatz von Phasentransferkatalysatoren, wie beispielsweise quartären Ammoniumsalzen, kann eine Beschleunigung der Reaktion erreicht werden. Als quartäre Ammoniumsalze kommen die oben bereits genannten in Frage.

Als Beispiele für die neuen Verbindungen der Formel IV seien im einzelnen genannt:
2,2-Dimethyl-3-(2',3',3'-trichlor-3'-fluor-propenyl)-cyclopropancarbonsäuremethylester,
2,2-Dimethyl-3-(2',3',3'-trichlor-3'-fluor-propenyl)-cyclopropancarbonsäureäthylester,
2,2-Dimethyl-3-(2',3',3'-trichlor-3'-fluor-propenyl)-cyclopropancarbonsäurebutylester,
1-Cyano-2,2-dimethyl-3-(2',3',3'-trichlor-3'-fluor-propenyl)-cyclopropan.

Die neuen Verbindungen der Formel IV lassen sich nach dem unter 6. (oben) angegebenen Verfahren aus den Verbindungen der Formel V durch Dehydrohalogenierung herstellen. Diese Reaktion lässt sich durch folgendes Formelschema darstellen:

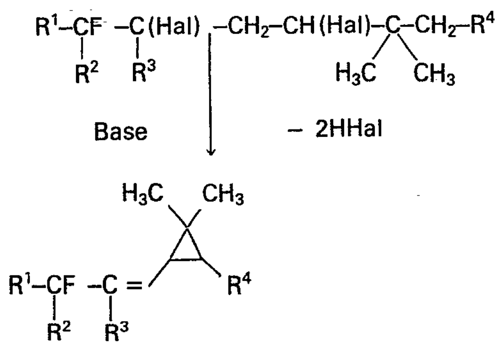


Die Dehydrohalogenierung erfolgt durch Basen in Anwesenheit von Verdünnungsmitteln. Als Basen kommen dabei in Frage:

$NaOH$, $KOH$, $K_2CO_3$, Alkoholate, wie Natriummethylat, Natriumisopropylat oder Kalium-tert.-butylat. Als Lösungsmittel kommen in erster Linie Alkohole in Frage, wie beispielsweise Methanol, Äthanol, n- oder iso-Propanol, Butanol, tert.-Butanol, Glykol oder Glykol-monomethyläther; ebenso aber auch gegebenenfalls chlorierte Kohlenwasserstoffe oder Äther, wie Benzol, Toluol, Xylol, Chlorbenzol, Dichlorbenzol, Methylenchlorid, THF, Dioxan, Dimethoxyäthan. Es kann auch, insbesondere bei Verwendung von NaOH oder KOH, in einem Zweiphasensystem – wie beispielsweise Toluol/Wasser gearbeitet werden. Gegebenenfalls kann ein Phasentransferkatalysator, wie beispielsweise ein quartäres Ammoniumsalz, zugefügt werden. Dies ist nicht nur beim Arbeiten im Zweiphasensystem sinnvoll, sondern auch beim wasserfreien Arbeiten von Vorteil, wenn beispielsweise Kaliumcarbonat als Base Verwendung findet.

Die Base, vorzugsweise $NaOCH_3$ oder $NaOC_2H_5$, wird in einem Lösungsmittel vorgelegt, dann die zu dehydrohalogenierende Verbindung der Formel V langsam zugefügt und zwar zunächst bei etwa 0 bis 30°C und dann bis zur Beendigung der Reaktion bei 40 bis 100°C, vorzugsweise bei 50 bis 80°C.

Als Beispiele für Verbindungen der Formel V seien genannt:
3,3-Dimethyl-4,6,6,7,7-pentachlor-7-fluor-önanthsäuremethylester,
3,3-Dimethyl-4,6,6,7,7-pentachlor-7-fluor-önanthsäureäthylester,
3,3-Dimethyl-4,6,6,7,7-pentachlor-7-fluor-önanthsäurebutylester,
3,3-Dimethyl-4,6,6,7,7-pentachlor-7-fluor-önanthsäurenitril,
2,2-Dimethyl-3,5,5,6,6-pentachlor-6-fluor-hexylmethylketon.

Die neuen Verbindungen der Formel V werden nach dem Verfahren 8. (oben) aus den Verbindungen der Formel VI und VII erhalten. Die Verbindungen der Formel VI und VII sind bekannt.

Das Verfahren wird durch folgendes Reaktionsschema wiedergegeben:

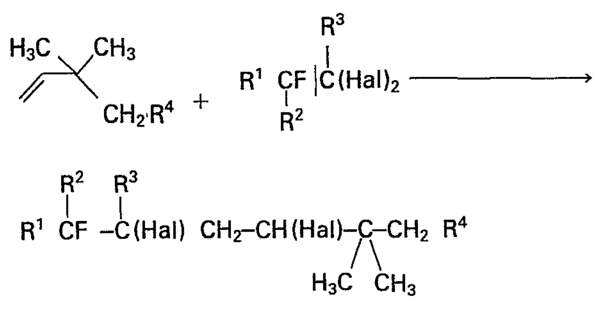

Verwendet man beispielsweise den 3,3-Dimethyl-4-pentensäure-methylester und 1-Fluor-1,1,2,2,2-pentachloräthan als Ausgangsstoffe, so lässt sich der Reaktionsverlauf durch folgendes Formelschema darstellen:

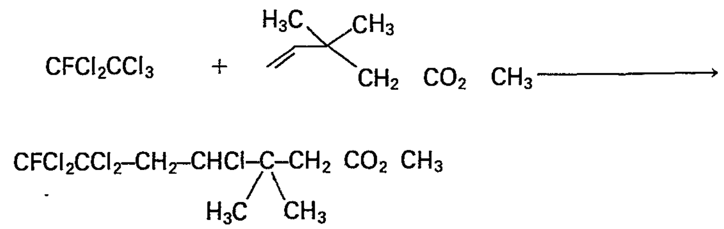

Folgende Ausgangsstoffe der Formel VI seien beispielsweise genannt:
1,2-Difluor-tetrachloräthan
1-Fluor-1,2,2-trichlor-1,2-dibromäthan
1,1,2,2-Tetrachlor-fluor-äthan

Folgende Ausgangsstoffe der Formel VII seien beispielsweise genannt:
3,3-Dimethyl-4-pentensäuremethylester
3,3-Dimethyl-4-pentensäureäthylester
3,3-Dimethyl-4-pentensäurebutylester
3,3-Dimethyl-4-pentensäurenitril
2,2-Dimethyl-3-butenyl-methylketon.

Das Verfahren wird vorzugsweise unter Druck, kann jedoch auch bei Verwendung von höhersiedenden Verbindungen der Formel VI bei Normaldruck durchgeführt werden.

Der Druckbereich kann in weiten Grenzen schwanken, etwa zwischen 1 und 30 bar, bevorzugt zwischen 3 und 15 bar. Der Überdruck kann durch Aufpressen eines Inertgases, beispielsweise von Stickstoff, erreicht werden.

Die Umsetzungstemperatur kann zwischen 50 und 200°C liegen, bevorzugt wird jedoch zwischen 100 und 150°C gearbeitet. Den beiden Ausgangsstoffen wird gegebenenfalls ein Verdünnungsmittel zugesetzt.

Als Verdünnungsmittel kommen in Frage: aliphatische oder aromatische Kohlenwasserstoffe, wie Benzin oder Toluol, Alkohole, wie Methanol Äthanol, Propanol oder tert.Butanol, Nitrile wie Acetonitril oder Propionitril, DMF. Bevorzugt sind Alkohole, Acetonitril und DMF.

Zur Durchführung der Reaktion sind Katalysatoren erforderlich. Und zwar entweder

a) freie Radikale liefernde Katalysatoren, wie z.B. Azobisisobutyronitril, Benzoylperoxid oder Di-tert.-butylperoxid.

Die Verbindungen der Formel VI werden äquimolar oder aber bevorzugt im Überschuss eingesetzt. Oder

b) Katalysatormischungen, die aus einem Metallsalz, einem Amin und gegebenenfalls etwas Benzoin bestehen. Das Metallsalz kann auch als Hydrat vorliegen, das Amin kann auch als Salz, z.B. als Hydrochlorid vorliegen.

Beispiele für Metallsalze sind:

Kupfer(I)chlorid, Kupfer(II)chlorid, Eisen(II)chlorid, Eisen(III)chlorid, Kupfer(II)sulfat, Kobaltchlorid, Zinkchlorid, Eisensulfat, Eisenacetat, Kupfer- oder Eisenacetylacetonat.

Beispiele für Amine sind:

Dimethylamin, Diäthylamin, Trimethylamin, Triäthylamin, Diisopropylamin, n-Butylamin, Benzylamin, Äthanolamin, Anilin, Pyridin. Bevorzugt sind Dimethylamin, Diäthylamin (als Hydrochloride), sowie n-Butylamin.

Es werden vorzugsweise mindestens 1,5 Mol, bevorzugt 2 bis 10 Mol organischen Amin pro Mol Metallsalz eingesetzt. Das Metallsalz wird in Mengen von 0,01 bis 15 Mol % vorzugsweise 0,05 bis 10 Mol % bezogen auf Verbindungen der Formel VII eingesetzt.

Die Zugabe von äquimolaren Mengen Benzoin bezogen auf das Metallsalz ist oft von Vorteil.

Dass die erfindungsgemässen Produkte der Formel VI, insbesondere das Pentachlorfluoräthan an die Olefine der Formel VII so gut addierbar sind, muss als äusserst überraschend bezeichnet werden und war vom Fachmann nicht zu erwarten, zumal sich Hexachloräthan nicht an Olefine addieren lässt.

Die Wirkstoffe eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z. B. Trichodectes spp., Damalinea spp.

Aus der Ordnung der Thysanoptera z. B. Hercinothrips femoralis, Thrips tabaci.

Aus der Ordnung der Heteroptera z. B. Eurygaster spp., Dysdercus intermedius, Piesma quadrata, Cimex lectularius, Rhodnius prolixus, Triatoma spp.

Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Marcrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z. B. Xenopsylla cheopis, Ceratophyllus spp.

Aus der Ordnung der Arachnida z. B. Scorpio maurus, Latrodectus mactans.

Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 Gewichtsprozent.

Die Anwendung der erfindungsgemässen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gewichtsprozent Wirkstoff, vorzugsweise zwischen 0,01 und 10 Gewichtsprozent liegen.

Die Anwendung geschieht in einer den Anwendungsformen angepassten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die Anwendung der erfindungsgemässen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgiessens (pour-on and spot-on) und des Einpuders sowie durch parenterale Anwendung in Form beispielsweise der Injektion.

Beispiel A

Phaedon-Larven-Test

Lösungsmittel: 3 Gewichtsteile Dimethylformamid

Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100%, dass alle Käfer-Larven abgetötet wurden; 0% bedeutet, dass keine Käfer-Larven abgetötet wurden.

Bei diesem Test zeigen z.B. die folgenden Verbindungen der Herstellungsbeispiele überlegene Wirksamkeit gegenüber dem Stand der Technik: 1

Beispiel B

Tetranychus-Test (resistent)

Lösungsmittel: 3 Gewichtsteile Dimethylformamid

Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, dass alle Spinnmilben abgetötet wurden; 0% bedeutet, dass keine Spinnmilben abgetötet wurden.

Bei diesem Test zeigen z.B. die folgenden Verbindungen der Herstellungsbeispiele überlegene Wirksamkeit gegenüber dem Stand der Technik: 1

Beispiel C

Grenzkonzentrations-Test/Bodeninsekten

Testinsekt: Tenebrio Molitor (im Boden)

Lösungsmittel: 3 Gewichtsteile Aceton

Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und lässt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100%, wenn alle Testinsekten abgetötet worden sind, er ist 0%, wenn noch genau so viele Testinsekten leben wie bei der unbehandelten Kontrolle.

Beispiel D

$LD_{100}$-Test

Testtiere: Blatta orientalis

Zahl der Testtiere: 10

Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschliessend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Ablösung in %. Dabei bedeutet 100%, dass alle Testtiere abgetötet wurden; 0% bedeutet, dass keine Testtiere abgetötet wurden.

Bei diesem Test zeigen z. B. die folgenden Verbindungen der Herstellungsbeispiele überlegene Wirkung gegenüber dem Stand der Technik: 1

Beispiel E
$LT_{100}$-Test für Dipteren
Testtiere: Aedes aegypti
Zahl der Testtiere: 25
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschliessend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100%igen knock down-Effekt notwendig ist.

Bei diesem Test zeigen z. B. die folgenden Verbindungen der Herstellungsbeispiele überlegene Wirkung gegenüber dem Stand der Technik: 2

Beispiel 1

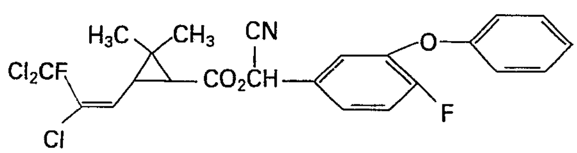

5,88 g 2,2-Dimethyl-3-(2′,3′,3′-trichlor-3′-fluorpropenyl)-cyclopropancarbonsäurechlorid werden mit 100 ml Toluol und 4,8 g Cyanhydrin des 3-Phenoxy-4-fluor-benzaldehyds vermischt. Dann tropft man bei Raumtemperatur unter Rühren 1,8 g Pyridin in 50 ml Toluol zu und rührt 4 h bei Raumtemperatur nach. Dann wird das Reaktionsgemisch mit 150 ml Wasser versetzt, die organische Phase abgetrennt und mit 100 ml Wasser gewaschen. Die organische Phase wird mit Natriumsulfat getrocknet, abfiltriert, einrottiert und bei 60°C im Hochvakuum abdestilliert (ca. 1 h). Der Rückstand wiegt 8,5 g und besteht aus 2,2-Dimethyl-3-(2′,3′,3′-trichlor-3′-fluorpropenyl)-cyclopropancarbonsäure-(3′-phenoxy-4′-fluor-α-cyano-)benzylester.

Analog Beispiel 1 wird folgende Verbindung hergestellt:

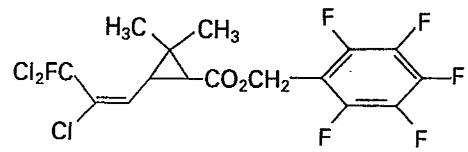

Beispiel 3
Herstellung von

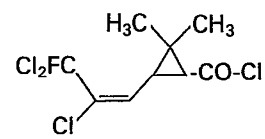

Man löst 20 g 2,2-Dimethyl-3-(2′,3′,3′-trichlor-3′-fluorpropenyl)-cyclopropancarbonsäure in 150 ml Methylenchlorid, gibt einige Tropfen DMF hinzu und leitet bei Raumtemperatur solange Phosgen ein, bis die Reaktion beendet ist. (Kontrolle durch das Infrarotspektrum). Anschliessend wird überschüssiges Phosgen und Methylenchlorid abdestilliert. Man erhält durch Destillation 2,2-Dimethyl-3-(2′,3′,3′-trichlor-3′-fluorpropenyl)-cyclopropancarbonsäurechlorid vom Siedepunkt 82–86°C/0,2 mm.

Beispiel 4
Herstellung von

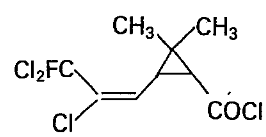

Man löst 20 g 2,2-Dimethyl-3-(2′-chlor-3′-trifluor-methyl-3′,4′,4′,4′-tetrafluor-but-1-enyl)-cyclopropancarbonsäure in 100 ml Thionylchlorid und erhitzt eine Stunde zum Sieden. Nach Abdestillieren des überschüssigen Thionylchlorids wird der Rückstand im Hochvakuum destilliert. Man erhält das 2,2-Dimethyl-3-(2′-chlor-3′-trifluormethyl-3′,4′,4′,4′-tetrafluorbut-1-enyl)-cyclopropancarbonsäurechlorid vom Siedepunkt 73-76°C/0,8 mm.

Beispiel 5

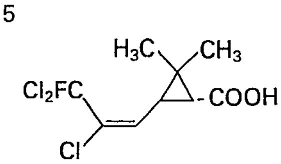

30,35 g 2,2-Dimethyl-3-(2',3',3'-trichlor-5'-fluorpropenyl)-cyclopropancarbonsäureäthylester werden mit 200 ml 3N methanolischer KOH versetzt und 6 h bei Raumtemperatur gerührt. Anschliessend versetzt man mit 300 ml Eiswasser und stellt mit konz. HCl auf pH 1. Durch Extrahieren mit Petroläther erhält man 23,5 g Carbonsäure, die nur teilweise erstarrt (Isomerengemisch).

Beispiel 6

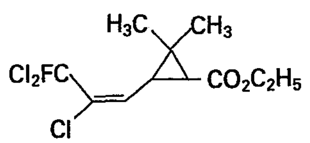

Zu einer Lösung von 16,1 g Natrium in 800 ml Äthanol werden 120 g 3,3-Dimethyl-4,6,6,7,7-pentachlor-7-fluor-önanthsäureäthylester getropft. Man rührt 1 h bei Raumtemperatur nach und erhitzt dann 7 h auf 45 bis 50°C. Nach dem Abkühlen filtriert man von abgeschiedenen Natriumchlorid ab, engt im Vakuum etwas ein, gibt auf 500 ml Wasser und stellt neutral. Durch Extrahieren mit Methylenchlorid und fraktionierte Destillation erhält man 82 g 2,2-Dimethyl-3-(2',3',3'-trichlor-3'-fluorpropenyl)-cyclopropancarbonsäureäthylester vom Siedepunkt 92 bis 97°C/0,3 mm.

Beispiel 7

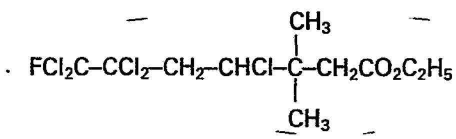

168 g 3,3-Dimethyl-4-pentensäureäthylester und 624 g Pentachlorfluoräthan werden zusammen mit 2,4 g Benzoylperoxid unter Rühren erhitzt. Bei einer Badtemperatur von 100°C steigt die Innentemperatur auf 118°C an. Nach ca. 2,5 h ist die Wärmetönung beendet.

Das überschüssige Pentachlorfluoräthan und der nicht umgesetzte 3,3-Dimethyl-4-pentensäureäthylester lassen sich leicht destillativ vom gebildeten 3,3-Dimethyl-4,6,6,7,7-pentachlor-7-fluorönanthsäureäthylester (Brechungsindex $n_D^{20}$ = 1,487) trennen.

Analog werden erhalten

Beispiel 8

| Verbindung | Kp (°C/mm) |
|---|---|
| 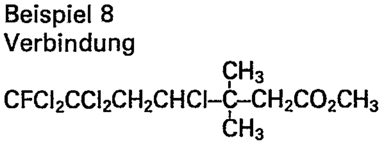 | 122–130/0,3 |

Beispiel 9

| Verbindung | Kp(°C/mm) |
|---|---|
| 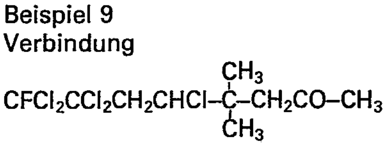 | 103–107/0,25 |

**Patentansprüche**

1) Fluoralkenylsubstituierte Cyclopropanverbindungen der Formel (I)

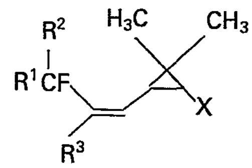

(I)

in welcher

$R^1$ für Chlor steht,

$R^2$ für Chlor steht,

$R^3$ für Cl oder Br,

X für $-\overset{O}{\overset{\|}{C}}$-Halogen, $-\overset{O}{\overset{\|}{C}}$-OH, $-\overset{O}{\overset{\|}{C}}$-O-R und

R für $C_{1-4}$-Alkyl sowie für die Reste der Formeln

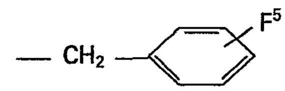

oder für den Fall, dass $R^3$ für Cl steht, für:

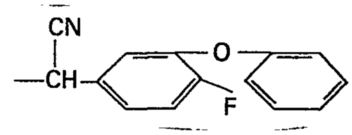

steht.

2) Verfahren zur Herstellung der fluoralkenylsubstituierten Cyclopropancarbonsäureester der Formel (I), dadurch gekennzeichnet, dass man eine Säure oder deren reaktionsfähiges Derivat der Formel (II)

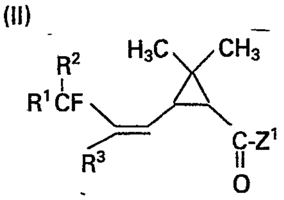

(II)

in welcher

$R^1$, $R^2$ und $R^3$ die unter Anspruch 1 angegebene Bedeutung haben und

$Z^1$ Halogen oder OH bedeutet,

mit einem Alkohol oder dessen reaktionsfähigem Derivat der Formel (III)

$$R - Z^2 \quad (III)$$

in welcher

R die in Anspruch 1 angegebene Bedeutung hat und

$Z^2$ OH, Cl oder Br bedeutet,

gegebenenfalls in Gegenwart von Lösungsmitteln, Säureakzeptoren und/oder Phasentransferkatalysatoren umsetzt, sowie für den Fall, dass

X für -CO-Halogen oder -COOH steht,

man die Verbindungen der Formel (IV)

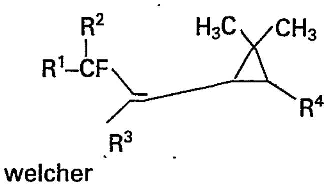

(IV)

in welcher

$R^4$ für $-COOC_{1-4}$-Alkyl, -CN oder $-COCH_3$ steht und

$R^1$, $R^2$ und $R^3$ die oben angegebene Bedeutung besitzen,

für den Fall, dass $R^4$ für $-\overset{O}{C}O-C_{1-4}$-Alkyl oder CN steht verseift oder für den Fall, dass $R^4$ für $-\overset{O}{C}-CH_3$ steht, oxidiert und gegebenenfalls die Säure, in welcher $R^4$ COOH bedeutet mit einem Halogenierungsmittel umsetzt, sowie für den Fall, dass

X für CN, $-\overset{O}{C}-CH_3$ oder $-\overset{O}{C}O-C_{1-4}$-Alkyl steht, dass man die Verbindungen der Formel (V)

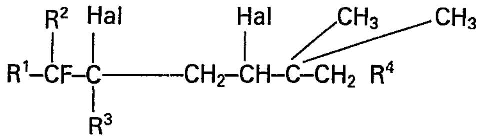

(V)

in welcher

$R^1$, $R^2$, $R^3$ und $R^4$ die oben angegebene Bedeutung besitzen, und

Hal für Halogen steht,

dehydrohalogeniert.

3) Insektizide und/oder akarizide Mittel, gekennzeichnet durch einen Gehalt an mindestens einem fluoralkenylsubstituierten Cyclopropancarbonsäureester der Formel (I) in welcher X für $-\overset{O}{C}-OR$ steht, wobei R die in Anspruch 1 angegebene Bedeutung hat aber nicht für $C_{1-4}$-Alkyl steht.

4) Verwendung von fluoralkenylsubstituierten Cyclopropancarbonsäureester der Formel (I) in welcher X für $-\overset{O}{C}-OR$ steht, wobei R die in Anspruch 1 angegebene Bedeutung hat aber nicht für $C_{1-4}$-Alkyl steht, zur Bekämpfung von Insekten und/oder Spinnentieren.

**Claims**

1) Fluoroalkenyl-substituted cyclopropane compounds of the formula (I)

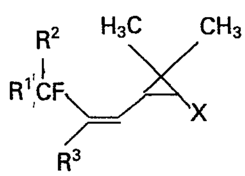

(I)

in which

$R^1$ represents chlorine,

$R^2$ represents chlorine,

$R^3$ represents Cl or Br,

X represents $-\overset{O}{C}$-halogen, $-\overset{O}{C}-OH$ or $-\overset{O}{C}-O-R$ and

R represents $C_{1-4}$-alkyl and the radicals of the formula

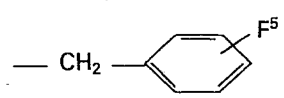

or in the case where $R^3$ represents Cl, represents

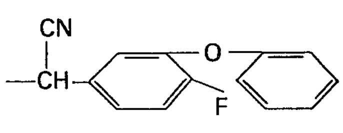

2) Process for the preparation of the fluoroalkenyl-substituted cyclopropanecarboxylic acid esters of the formula (I), characterised in that an acid or reacitve derivative thereof, of the formula (II)

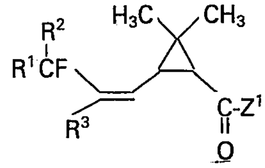

(II)

in which

$R^1$, $R^2$ and $R^3$ have the meaning indicated under Claim 1 and

$Z^1$ denotes halogen or OH,

is reacted with an alcohol or reactive derivative thereof, of the formula (III)

$$R - Z^2 \qquad \text{(III)}$$

in which

R has the meaning indicated in Claim 1 and

$Z^2$ denotes OH, Cl or Br,

if appropriate in the presence of solvents, acid acceptors and/or phase transfer catalysts, and in the cas where

X represents -CO-halogen or -COOH,

the compounds of the formula (IV)

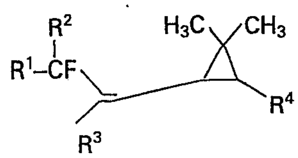

(IV)

in which

$R^4$ represents $-COOC_{1-4}$-alkyl, -CN or $-COCH_3$ and

$R^1$, $R^2$ and $R^3$ have the meaning indicated above,

are saponified in the case where

$R^4$ represents $-\overset{O}{C}O-C_{1-4}$-alkyl or CN,

or are oxidised, in the case where

$R^4$ represents $-\overset{O}{C}-CH_3$, and,

if appropriate, the acid in which $R^4$ denotes COOH is reacted with a halogenating agent, and in the case where

X represents CN, $-\overset{O}{C}-CH_3$ or $-\overset{O}{C}O-C_{1-4}$-alkyl,

in that the compounds of the formula (V)

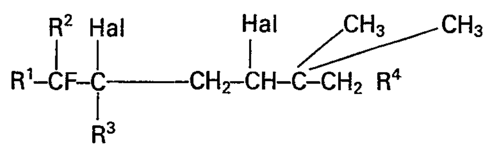

(V)

in which

$R_1$, $R^2$, $R^3$ and $R^4$ have the meaning indicated above and

Hal represents halogen,

are dehydrohalogenated.

3) Insecticidal and/or acaricidal agents, characterised in that they contain at least one fluoroalkenyl-substituted cyclopropanecarboxylic acid ester of the formula (I)

in which

X represents -$\overset{O}{\overset{\|}{C}}$-OR,
wherein R has the meaning indicated in Claim 1 but does not represent $C_{1\text{-}4}$-alkyl.

4) Use of fluoroalkenyl-substituted cyclopropanecarboxylic acid esters of the formula (I) in which

X represents -$\overset{O}{\overset{\|}{C}}$-OR,
wherein R has the meaning indicated in Claim 1 but does not represent $C_{1\text{-}4}$-alkyl, for combating insects and/or arachnidae.

**Revendications**

1) Dérivés du cyclopropane portant des substituants fluoralcényle et répondant à la formule (I)

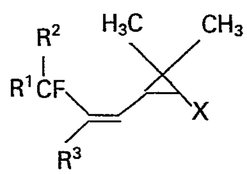

(I)

dans laquelle
$R^1$ représente le chlore,
$R^2$ représente le chlore,
$R^3$ représente Cl ou Br,
X représente -$\overset{O}{\overset{\|}{C}}$-halogène, -$\overset{O}{\overset{\|}{C}}$-OH, -$\overset{O}{\overset{\|}{C}}$-O-R, et
R représente un groupe alkyle en C1–C4 ou des restes de formules

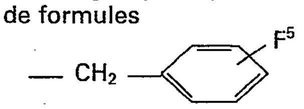

ou bien, dans le cas où $R^3$ représente Cl,

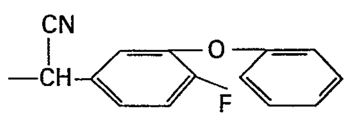

2) Procédé de préparation des esters cyclopropane-carboxyliques à substituants fluoralcényle de formule (I), caractérisé en ce que l'on fait réagir un acide ou son dérivé réactif de formule (II)

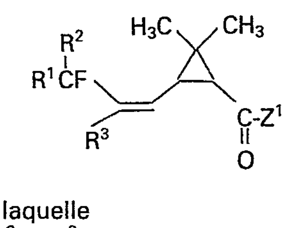

(II)

dans laquelle
$R^1$, $R^2$ et $R^3$ ont les significations indiquées dans la revendication 1, et
$Z^1$ représente un halogène ou un groupe OH,
avec un alcool ou son dérivé réactif de formule (III)

$$R - Z^2 \qquad (III)$$

dans laquelle
R a la signification indiquée dans la revendication 1, et
$Z^2$ représente OH, Cl ou Br,
éventuellement en présence de solvants, d'accepteurs d'acides et/ou de catalyseurs à transfert de phase et, dans le cas où
X représente -CO-halogène ou -COOH,
on saponifie les composés de formule (IV)

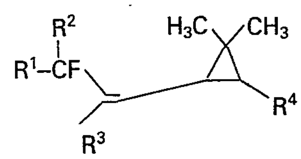

(IV)

dans laquelle
$R^4$ représente -COO-alkyle en C1–C4, -CN ou $-COCH_3$ et
$R^1$, $R^2$ et $R^3$ ont les significations indiquées ci-dessus,

dans le cas où $R^4$ représente -$\overset{O}{\overset{\|}{C}}$O-alkyle en C1–C4 ou CN, ou bien dans le cas où $R^4$ représente -$\overset{O}{\overset{\|}{C}}$-$CH_3$, on oxyde les composés de formule IV et, le cas échéant, on fait réagir l'acide dans lequel $R^4$ représente COOH avec un agent halogénant et dans le cas où

X représente CN, -$\overset{O}{\overset{\|}{C}}$-$CH_3$ ou -CO-alkyle en $\overset{O}{\overset{\|}{C}}$1–C4,
on soumet les composés de formule V

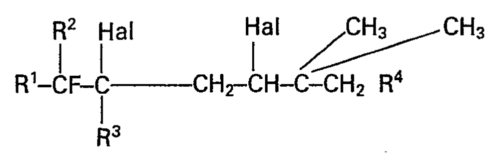

(V)

dans laquelle,
$R_1$, $R^2$, $R^3$ et $R^4$ ont les significations indiquées ci-dessus, et
Hal représente halogène,
à déshydrohalogénation.

3) Produits insecticides et acaricides, caractérisés en ce qu'ils contiennent au moins un ester cyclopropane-carboxylique à substituants fluoralcényle de formule (I) dans laquelle X représente

-$\overset{O}{\overset{\|}{C}}$-OR, R ayant la signification indiquée dans la revendication 1 à l'exception d'un groupe alkyle en C1–C4.

4) Utilisation des esters cyclopropane-carboxyliques à substituants fluoralcényle de formule (I)

dans laquelle X représente-$\overset{O}{\overset{\|}{C}}$-OR, dans laquelle R a la signification indiquée dans la revendication 1, à l'exception d'un groupe alkyle en C1–C4, dans la lutte contre les insectes et/ou les acariens.